# EUROPEAN PATENT APPLICATION

(11) **EP 4 606 369 A1**
(43) Date of publication of application: **27.08.2025**
(21) Application number: 23893650.4
(22) Date of filing: 10.11.2023
(51) Int. Cl.: A61K 9/127, A23L 33/10, A23P 10/30, A61K 31/047, A61K 31/12

(54) **HIGH-STABILITY LIPOSOME, PREPARATION METHOD THEREFOR, AND USE THEREOF**

(30) Priority: 25.11.2022 CN 202211486759
(71) Applicant: Sirio Healthcare (Anhui) Co., Ltd, Ma'anshan, Anhui 243099 (CN)
(72) Inventor: WANG, Peijian, Shantou, Guangdong 515000 (CN); FANG, Suqiong, Shantou, Guangdong 515000 (CN); CHEN, Wenrong, Shantou, Guangdong 515000 (CN)
(74) Representative: Cabinet Becker et Associés
(86) International application number: PCT/CN2023/130881
(87) International publication number: WO 2024/109560

(57) **Abstract**

Provided are a high-stability liposome, a preparation method therefor, and use thereof. A liposome composition comprises: 2-5 parts by weight of liquid oil, 3-10 parts by weight of solid oil, 5-30 parts by weight of phospholipid, 3-10 parts by weight of gelatin, 1-7 parts by weight of active ingredients, and 20-70 parts by weight of water. The liquid oil is one or more of glyceryl caprylate-caprate, soybean oil, and sunflower seed oil. The solid oil is one or more of glyceryl mono- and distearate, citrate, palm stearin, and beewax. The phospholipid is phospholipid with a phosphatidylcholine content of 30 wt%-60 wt%. The gelatin is gelatin with a Bloom value of 200-300. The liposome composition does not comprise an excipient or comprises 20-70 parts by weight of an excipient. The liposome herein does not comprise cholesterol, and has the characteristics of high temperature resistance and high stability.

## Description

This application claims the priority of the Chinese invention patent application No. 202211486759.6, filed on November 25, 2022, and titled "HIGH-STABILITY LIPOSOME, PREPARATION METHOD THEREFOR, AND USE THEREOF".

### Technical Field

The disclosure relates to the field of a formulation and application thereof, in particular to a liposome formulation and preparation methods therefor.

### Background of the Disclosure

A liposome is a small vesicle formed by encapsulating active ingredients (such as drugs) within a bilayer structure that mimics the lipid bilayer. Conventional liposomes are composed of phospholipids and cholesterol, featuring an internal vesicular structure where the lipid bilayer encapsulates an aqueous phase. As a new-generation drug-delivery system, liposomes possess properties such as enhancing drug efficacy, reducing toxicity, and enabling long-circulating and targeting effects.

Liposomes can be prepared via various methods. According to different drug-loading mechanisms, these methods are classified into active drug-loading methods and passive drug-loading methods. Active drug-loading methods involve first preparing blank liposomes and then loading drugs through ion or compound gradients between the internal and external aqueous phases of the liposomes. This type of method is often used for amphiphilic substances. Passive drug-loading methods involve dissolving the drug in an aqueous phase (for water-soluble drugs) or an organic phase (for fat-soluble drugs) beforehand, followed by preparing drug-loaded liposomes using a selected liposome preparation method.

Chinese Patent CN109463751B discloses a coenzyme Q10 liposome health food and its preparation method. In this method, coenzyme Q10, lecithin, cholesterol, Tween-80, and VE acetate are dissolved in ethanol under heating, followed by reduced-pressure evaporation to form a smooth film. After vacuum drying, polyvinylpyrrolidone and glycerol are added, and an aqueous medium is introduced to hydrate the film, yielding an emulsified liquid. Ultrasonication of the emulsified liquid produces coenzyme Q10 liposomes with nanometer-scale particle sizes. This method achieves a 60% encapsulation efficiency for coenzyme Q10 liposomes, with bioaccessibility threefold that of the bulk drug. However, the use of Tween and cholesterol as excipients in CN109463751B limits its applicability in food and health food products. Additionally, organic solvents are employed in the production process, and the liposome products exhibit relatively low encapsulation efficiency (only 60%) and bioaccessibility (merely threefold that of the bulk drug).

The Chinese patent CN103989635B discloses a method for preparing a coenzyme Q10 nanoliposome by supercritical carbon dioxide, which comprises the following steps: adding phosphatidylcholine, cholesterol and coenzyme Q10 into absolute ethanol, fully dissolving them while stirring to prepare a solution, and putting the solution into an ultrasonic shaker for mixing them; putting the prepared solution into a supercritical reactor, opening a carbon dioxide cylinder, cooling the carbon dioxide gas into a liquid by a cooling machine, followed by pressurizing the carbon dioxide liquid by a high-pressure pump, and then introducing the carbon dioxide liquid into the reactor, and controlling the pressure and temperature, and maintaining the pressure for a period of time; quickly spraying the solution into a phosphate buffer solution through a nozzle, dispersing and precipitating to obtain a coenzyme Q10 nanoliposome suspension; and collecting a product, i.e., coenzyme Q10 nanoliposomes. The encapsulation efficiency of this coenzyme Q10 liposome is 78%. Cholesterol and ethanol are also used in the liposome technology of the patent CN103989635B. Taking too much cholesterol is disadvantageous for health, and it is in great danger when organic solvents are used in the production process.

Russian patent RU2605616C1 discloses a liposome based on UBIQUINOL and preparation method therefor. The method involves preparing a solution containing a phospholipid and sterol mixture at a mass ratio of 1:0.1-2, respectively; ubiquinol at a mass ratio of ubiquinol to the phospholipid and sterol mixture of 1:1-4, respectively; and a cosolvent comprising a nonionic surfactant in ethanol. It also involves dispersing the obtained solution containing the lipid fraction in an aqueous medium with a cryoprotectant to form a suspension of lipid vesicles encapsulated with ubiquinol, homogenizing the lipid vesicle suspension, and separating liposomes with a size of no more than 220 nm containing ubiquinol. Tween and ethanol are also used in the patent RU2605616C1. Tween is harmful to humans with enteritis and gastrointestinal sensitivity. Using ethanol during the production is also dangerous.

The Chinese patent CN105030679A discloses a high-stability nanoliposome antibacterial agent of sage essential oil and its preparation method. In the patent, the nanoliposome of sage essential oil is made from sage essential oil, soybean lecithin, cholesterol, a surfactant, chitosan and gelatin. The method comprises the following steps: mixing sage essential oil, soybean lecithin and cholesterol in an organic solvent, evaporating to dryness under reduced pressure to form a smooth film, adding an aqueous medium and a surfactant to dissolve the film and forming an emulsion by ultrasonication, mixing with chitosan and gelatin solution while stirring to homogeneity, and centrifuging and filtering with a microporous filter membrane to obtain liposomes with nanometer particle size. Cholesterol and surfactants are used in the excipients of the patent CN105030679A, and organic solvents are also used in the preparation process, thus bringing about certain limitations in application in the field of food and health food.

There is still a need in the art for highly stable liposome preparations for the delivery of active ingredients.

### Summary of the Disclosure

To address the deficiencies in the prior art, the present disclosure prepares a liposome without cholesterol by using a phospholipid with a specific phosphatidylcholine content range (PC content of 30 - 60 wt%) in combination with gelatin with a high Bloom value (200-300). The prepared liposomes may have one or more of the following properties: high temperature resistance, high stability, high encapsulation efficiency, high retention rate, and bioaccessibility.

In one aspect, the present disclosure provides a liposome composition comprising 2 to 5 parts by weight of a liquid oil, 3 to 10 parts by weight of a solid oil, 5 to 30 parts by weight of a phospholipid, 3 to 10 parts by weight of gelatin, 1 to 7 parts by weight of an active ingredient, and 20 to 70 parts by weight of water.

In one embodiment, the liquid oil is one or more of glyceryl caprylate-caprate, soybean oil, and sunflower seed oil. In one embodiment, the solid oil is one or more of glyceryl mono- and distearate, citrate, palm stearin, and beeswax. In one embodiment, the phospholipid is phospholipid with a phosphatidylcholine content of 30 wt% to 60 wt%. In one embodiment, the gelatin is gelatin with a Bloom value between 200 and 300. In one embodiment, the liposome composition does not comprise an excipient. In one embodiment, the liposome composition comprises 20 to 70 parts by weight of an excipient.

In one embodiment, the active ingredient is selected from the group consisting of a fat-soluble active ingredient, a water-soluble active ingredient, a heat-sensitive active ingredient, an insoluble particle, an acid-sensitive active ingredient, or a digestive enzyme-sensitive active ingredient. In one embodiment, the active ingredient is a fat-soluble or water-soluble heat-sensitive active ingredient. In one embodiment, the active ingredient is an insoluble particle.

In one embodiment, the active ingredient is one or more of coenzyme Q10, curcumin, lutein and astaxanthin.

In one embodiment, the liposome composition does not comprise one or more of the following ingredients: cholesterol, ethanol, and Tween. In one embodiment, the excipient is one or more of a colloid, a maltodextrin, a cyclodextrin, a starch, a sugar alcohol, a syrup, and a whey protein.

In one aspect, the present disclosure provides a method of preparing a liposome described herein, comprising mixing the liquid oil, the solid oil, the phospholipid, gelatin, the active ingredient and water to form a liposome fluid.

In one embodiment, when the fat-soluble active ingredient is used, the method comprises:
(1) mixing homogeneously the fat-soluble active ingredient, the liquid oil and the solid oil to obtain a component labeled as A1;
(2) mixing homogeneously the phospholipid, the gelatin and water to obtain a component labeled as B1; and
(3) mixing the component A1 and the component B1 to form a homogeneous liquid system.

In one embodiment, when the water-soluble active ingredient is used, the method comprises:
(1) mixing homogeneously the liquid oil and the solid oil to obtain a component labeled as A2;
(2) mixing homogeneously the water-soluble active ingredient, the phospholipid, the gelatin and water to obtain a component labeled as B2; and
(3) mixing the component A2 and the component B2 to form a homogeneous liquid system.

In one aspect, the present disclosure provides a method of preparing a dosage form of a liposome, comprising the method of preparing the liposome described herein, and further comprises one or more of the following steps:
thermally sterilizing the liposome liquid at a high temperature to prepare a sterilized liquid liposome;
adding a colloid excipient to the liposome liquid, and sufficiently dispersing the colloid excipient, and thermally sterilizing the mixture at a high temperature to obtain a liposome gel; or
adding an excipient to the liposome liquid, followed by mixing, thermally sterilizing at a high temperature, and drying to obtain a liposome powder.

In one embodiment, the excipient is one or more of maltodextrin, cyclodextrin, starch, sugar alcohol, syrup, and whey proteins.

In one aspect, the present disclosure provides a liposome fluid prepared by the method of preparing the liposome described herein.

In one aspect, the present disclosure provides a dosage form of a liposome prepared from the liposome fluid described herein. In one embodiment, the dosage form of the liposome is a solution, a gel, a powder, a tablet, a soft capsule, or a gummy.

In one aspect, the disclosure provides a dosage form of the liposome that is a liquid liposome, a liposome gel, or a liposome powder. In one embodiment, the dosage form of the liposome is prepared by the method described herein.

In one aspect, the present disclosure provides the use of the dosage form of liposome described herein in a food or health products or in the manufacture of a pharmaceutical.

The present disclosure provides the following beneficial effects:
1. The liposome described herein contains no cholesterol, is resistant to high temperatures, and has high stability.
2. The liposome of the present disclosure provides high encapsulation efficiency, high retention rate, and high bioaccessibility for the active ingredients described herein (e.g., coenzyme Q10, etc.).
3. The food raw materials and excipients employed in this disclosure exhibit high safety, broad application scope, and advantages in industrial production and cost savings.
4. Compared with the prior art, the liposome product of the active ingredient (e.g., coenzyme Q10) in this disclosure exhibits excellent stability, no stratification, and small uniform particle size. After three months of accelerated stability testing (40°C, 70% humidity), its encapsulation efficiency remains above 92%, and the active ingredient retention rate exceeds 93%. Notably, its bioaccessibility is significantly higher than that of the raw materials. The excipients selected for the composition are safe, and no organic solvents are used in the preparation process.
5. The powder of the present disclosure has excellent redissolvability.

### Brief Description of the Drawings

Fig. 1 shows a comparison graph of the precipitation or stratification degrees after high-temperature sterilization of coenzyme Q10 liposome samples.
Fig. 2 shows the coenzyme Q10 raw material with a content of 98%. The structures of coenzyme Q10 raw material under the microscope show irregular crystalline structures.
Fig. 3 shows a micrograph of the redissolved solution of Composition 12 in Table 3.
Fig. 4 shows the scanning electron microscopy (SEM) structure of the sample of Composition 6 in Table 2.
Fig. 5 shows a scanning electron microscopy image of the coenzyme Q10 liposome powder of Composition 12 in Table 3.
Fig. 6 shows the appearance of the coenzyme Q10 liposome solution of Composition 6 in Table 2.
Fig. 7 shows the appearance of the coenzyme Q10 liposome powder of Composition 12 in Table 3.
Fig. 8 shows the appearance of the redissolved solution of the coenzyme Q10 liposome powder of Composition 12 in Table 3.

### Detailed Description of the Disclosure

### Definition

In order to facilitate the understanding of the present disclosure, a number of terms are defined below. The terms defined herein have the meanings commonly understood by one of ordinary skill in the art to which the present disclosure relates.

As used herein, "liposomes" are tiny vesicles formed by encapsulating an active ingredient, such as a drug, in bilayer, mimicking a lipid bilayer. Conventional liposomes are composed of phospholipids and cholesterol and contain internal vesicle structures with a lipid bilayer encapsulating an aqueous phase.

As used herein, a "liposome composition" refers to raw materials used to prepare liposomes by conventional liposome preparation methods (e.g., passive drug loading).

As used herein, "Passive drug loading" involves firstly dissolving a drug in an aqueous phase (for a water-soluble drug) or an organic phase (for a fat-soluble drug), and then preparing a drug-containing liposome according to the selected liposome preparation method.

As used herein, a "liquid oil" refers to an oil that is liquid at room temperature.

As used herein, a "solid oil" refers to an oil that is solid at room temperature.

As used herein, "Bloom value" (or "Bloom Strength") is an important physical property of gelatin. Bloom value of gelatin is measured using the method specified in GB 6783-94 in China.

As used herein, an "active ingredient" is synonymous with "functional ingredient." In one embodiment, the active ingredient is selected from the group comprising fat-soluble active ingredients, water-soluble active ingredients, heat-sensitive active ingredients, insoluble particles, acid-sensitive active ingredients, or digestive enzyme-sensitive active ingredients, provided that it can be encapsulated to form a liposome. The active ingredient may be fat-soluble or water-soluble, or exist in the form of insoluble particles. The insoluble particles may have a size of 1 µm to 10 mm, such as 10 µm, 50 µm, 100 µm, 150 µm, 200 µm, 250 µm, 300 µm, 350 µm, 400 µm, 450 µm, 500 µm, 550 µm, 600 µm, 650 µm, 700 µm, 800 µm, 850 µm, 900 µm, 950 µm, 1 mm, 1.5 mm, 2 mm, 3 mm, 4 mm, 5 mm, 6 mm, 7 mm, 8 mm, or 9 mm. The particle size may be defined as the longest dimension of the particle. The particle shape is not particularly limited and may include various regular or irregular forms. Preferably, when the active ingredient is thermosensitive, it can withstand heat treatment after being encapsulated by the liposomes. Similarly, where the active ingredient is acid-sensitive or digestive enzyme-sensitive, it can resist digestion by digestive juices (e.g., gastric fluid) following liposomal encapsulation. Exemplary active ingredients in this context include coenzyme Q10 and/or curcumin. As used herein, "coenzyme Q10" refers to a fat-soluble antioxidant capable of activating human cells and cellular energy. It exhibits functions such as enhancing human immunity, boosting antioxidation, delaying aging, and improving human vitality. In the adjuvant treatment of cardio-cerebrovascular diseases and other conditions, coenzyme Q10 prevents cellular damage and demonstrates significant efficacy in enhancing immunity. However, coenzyme Q10 is heat-sensitive: conventional processing methods like sterilization and spray drying often cause its degradation due to temperature exposure. Additionally, coenzyme Q10 liposomes typically employ phospholipids as membrane materials, which are susceptible to structural damage by gastrointestinal acids, alkalis, and enzymes upon oral administration-leading to easy leakage of the active ingredient. Consequently, existing coenzyme Q10 liposome products suffer from low stability and bioaccessibility. The liposomes disclosed herein are exemplified in the Examples using coenzyme Q10, providing a formulation with high stability and bioaccessibility.

### Liposome Composition

Provided herein is a liposome composition comprising a liquid oil, a solid oil, a phospholipid, gelatin, an active ingredient, water, and optionally an excipient. The liposome composition of this disclosure may exclude one or more of the following ingredients: sterols, cholesterol, ethanol, Tween, and other organic solvents. Alternatively, the liposome composition is prepared without using one or more of the following: sterols, cholesterol, ethanol, Tween, and other organic solvents. Excessive intake of sterols or cholesterol is detrimental to health. The use of organic solvents in the production process possesses significant safety hazards. Tween is harmful to individuals with enteritis or gastrointestinal sensitivity. The liposome compositions described herein may consist of a liquid oil, a solid oil, a phospholipid, gelatin, an active ingredient, and water, or may consist of a liquid oil, a solid oil, a phospholipid, gelatin, an active ingredient, water, and an excipient.

In the liposome composition herein, the liquid oil is one or more of glyceryl caprylate-caprate, soybean oil, and sunflower seed oil. A liposome composition herein may comprise 2 to 5 parts by weight of a liquid oil, for example, 2, 2.5, 3, 3.5, 4, 4.5, or 5 parts by weight of a liquid oil.

In the liposome composition herein, the solid oil is one or more of glyceryl mono- and distearate, citrate, palm stearin, and beeswax. The liposome composition herein may comprise 3 to 10 parts by weight of a solid oil, for example, 4, 5, 6, 7, 8, or 9 parts by weight of a solid oil.

In the liposome composition herein, the phospholipid is a phospholipid with a phosphatidylcholine content of 30 to 60 wt%. The content of phosphatidylcholine may be 31 wt%, 32 wt%, 33 wt%, 34 wt%, 35 wt%, 36 wt%, 37 wt%, 38 wt%, 39 wt%, 40 wt%, 41 wt%, 42 wt%, 43 wt%, 44 wt%, 45 wt%, 46 wt%, 47 wt%, 48 wt%, 49 wt%, 50 wt%, 51 wt%, 52 wt%, 53 wt%, 54 wt%, 55 wt%, 56 wt%, 57 wt%, 58 wt%, or 59 wt%. The liposome composition herein may comprise 5 to 30 parts by weight of a phospholipid, such as 6, 7, 8, 9, 10, 11, 12, 13, 14, 15, 16, 17, 18, 19, 20, 21, 22, 23, 24, 25, 26, 27, 28, or 29 parts by weight of a phospholipid.

In the liposome composition herein, the gelatin is gelatin with a Bloom value of 200 to 300. For example, the gelatin has a Bloom value of 210, 220, 230, 240, 250, 260, 270, 280, or 290. The liposome composition herein may comprise 3 to 10 parts by weight of gelatin, for example, 4, 5, 6, 7, 8, or 9 parts by weight of gelatin.

In the liposome composition herein, the liposome composition may not comprise an excipient or may comprise 20 to 70 parts by weight of an excipient, for example, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, or 69 parts by weight of an excipient. The excipient may be one or more of a colloid, maltodextrin, cyclodextrin, starch, sugar alcohol, syrup, and whey protein. For example, the excipient is maltodextrin and isolated whey protein.

In the liposome composition herein, the liposome composition may comprise 1 to 7 parts by weight of the active ingredient, for example, 2, 3, 4, 5, or 6 parts by weight of the active ingredient. The active ingredient may be fat-soluble or water-soluble. The active ingredient may be a heat-sensitive active ingredient. For example, the active ingredient is coenzyme Q10.

In a liposome composition herein, the liposome composition may comprise 20 to 70 parts by weight of water, for example, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, or 69 parts by weight of water.

### Method for preparing liposomes

The present disclosure provides a method for preparing liposomes. The method may be a passive drug loading method. The method of the present disclosure may comprise mixing the liquid oil, the solid oil, the phospholipid, the gelatin, the active ingredient and water to form a liposome fluid. In one embodiment, when the fat-soluble active ingredient is used, the method comprises: (1) mixing homogeneously the fat-soluble active ingredient, the liquid oil and the solid oil to obtain a component labeled as A1;(2) mixing homogeneously the phospholipid, the gelatin and water to obtain a component labeled as B1; and (3) mixing the component A1 and the component B 1 to form a homogeneous liquid system. In one embodiment, when the water-soluble active ingredient is used, the method comprises: (1) mixing homogeneously the liquid oil and the solid oil to obtain a component labeled as A2; (2) mixing homogeneously the water-soluble active ingredient, the phospholipid, the gelatin and water to obtain a component labeled as B2; and (3) mixing the component A2 and the component B2 to form a homogeneous liquid system.

For example, the method of the present disclosure may comprise the following steps:
(1) fully dissolving and homogeneously mixing the coenzyme Q10, glyceryl caprylate-caprate, glyceryl mono- and distearate to obtain a component labeled as A;
(2) fully dissolving the phospholipid and gelatin in water and homogeneously mixing to obtain a component labeled as B; and
(3) pouring the component A into the component B, stirring, mixing to form a homogeneous system, and then homogenizing (e.g., twice at 600 bar).

The present disclosure also provides a method of preparing a dosage form of a liposome, which comprises the method of preparing the liposome described above, and further comprises one or more of the following steps:
thermally sterilizing the liposome liquid at a high temperature to prepare a sterilized liquid liposome;
adding a colloid excipient to the liposome liquid, and sufficiently dispersing the colloid excipient, and thermally sterilizing the mixture at a high temperature to obtain a liposome gel; or
adding an excipient to the liposome liquid, followed by mixing, thermally sterilizing at a high temperature, and drying to obtain a liposome powder. The excipient may be one or more of a maltodextrin, a cyclodextrin, a starch, a sugar alcohol, a syrup, and a whey protein.

For example, a method for preparing a dosage form of a liposome may comprise:
(1) fully dissolving and homogeneously mixing the coenzyme Q10, glyceryl caprylate-caprate, glyceryl mono- and di-stearate to obtain a component labeled as A;
(2) fully dissolving the phospholipid and gelatin in water and homogeneously mixing to obtain a component labeled as B;
(3) pouring the component A into the component B while stirring to form a homogeneous system, and then homogenizing (e.g., twice at 600 bar).

A liposome solution can be prepared by directly thermally sterilizing the homogenized solution obtained in the step (3) at a high temperature (for example, at 80-100°C for 10-30 minutes). A coenzyme Q10 liposome gel can be obtained by adding 20 to 70 parts of an excipient, preferably a colloid, to the homogenized solution in the step (3), then sufficiently dispersing it by means of high shear, colloid milling, high pressure homogenization, etc., and thermally sterilizing at a high temperature (for example, at 80 to 100°C for 10 to 30 min). A coenzyme Q10 liposome powder can be obtained by adding 20 to 70 parts of excipients (preferably, a maltodextrin, a cyclodextrin, a starch, a sugar alcohol, a syrup, a whey protein, and etc.) into the homogenized solution in the step (3), and homogenizing the excipients, followed by thermal sterilization at a high temperature (for example, at 80 to 100°C for 10 to 30 mins), and finally, removing the moisture by spray drying, freeze drying, and the like.

### Liposomes and dosage forms thereof

The liposomes herein can be directly used in the form of the liposome fluid prepared above, or in a specific dosage form prepared by adding an excipient to the liposome fluid. The dosage form of liposome may be a solution, a gel, a powder, a tablet, a soft capsule, or a gummy. The liposome fluid may be sterilized (e.g., thermally sterilized at a high temperature) or unsterilized. Preferably, the liposome fluid may be thermally sterilized at a high temperature. Preferably, the dosage form of liposome herein is a liquid liposome, a liposome gel or a liposome powder prepared by the method described herein. For example, the coenzyme Q10 liposome powder can be prepared by spray drying, after adding 20 to 70 parts of excipients to the coenzyme Q10 liposome described herein. The content of the coenzyme Q10 liposome in the powder is in the range of 0.8% to 18%. As compared with the liquid preparation, the powder has higher stability, which is beneficial to storage, and has a broader application. It can be directly used as a powder for making a solution, and can also be applied to health foods, for example, in the form of a tablet, a granule, a soft capsule, and a gummy.

### Application

The liposome dosage form described herein may be used in foods or health products or in the manufacture of pharmaceuticals. Taking coenzyme Q10 as an example of the active ingredient, coenzyme Q10 can prevent cell damage and has a good effect on improving human immunity in the adjuvant treatment of diseases such as heart and brain diseases. The specific functions are as follows:
1. Improvement and prevention of heart diseases: The heart is a high-energy-consuming organ, and its coenzyme Q10 content is the highest among all organs in the human body. When the body is deficient in coenzyme Q10, the heart is the first to be affected. Coenzyme Q10 can enhance myocardial function and improve cardiovascular diseases.
2. Protection of brain nerve cells: Apart from the heart, the brain is the most active organ in the human body with high energy demands. Coenzyme Q10 can provide sufficient oxygen and energy for brain cells and maintain healthy and active brain and nerve cells.
3. Antioxidant effects and reduction of apoptosis: Coenzyme Q10 has antioxidant effects, which can scavenge free radicals, prevent cells from damage, reduce apoptosis, and protect cells. The aging of the skin and the increase in wrinkles are also related to the Q10 content. Coenzyme Q10 can increase the concentration of hyaluronic acid in the skin, improve skin moisture content, and has a good effect on improving dull skin tone and reducing wrinkles.
4. Enhancement of body immunity: Coenzyme Q10 has the effect of scavenging free radicals, and its antioxidant capacity is 50 times that of vitamin E. Coenzyme Q10 can enhance the immune system's ability and play a good role in improving body immunity, anti-inflammation, and anti-tumor effects. It can also reduce the discomfort caused by radiotherapy and chemotherapy in cancer patients.
5. Adjuvant treatment of other diseases: Supplementing coenzyme Q10 has adjuvant treatment effects on migraines, emphysema, facial nerve palsy, periodontitis, alopecia areata, chronic obstructive pulmonary disease, bronchitis, and asthma. Clinical observations have also shown that muscular dystrophy, kidney diseases, etc., are related to coenzyme Q10 deficiency.

Hereinafter, embodiments of the present application will be described in detail in conjunction with Examples. Those skilled in the art will understand that the following Examples are merely for illustrating the present application and should not be regarded as limiting the scope of the present application. If specific conditions are not specified in an Example, conventional conditions or conditions recommended by the manufacturer shall be followed. Reagents and instruments used without indicating the manufacturer are all conventional commercially available products. Unless otherwise indicated, the amounts listed are based on the total weight and are described in parts by weight. This application should not be construed as being limited to the specific Examples described.

### Examples

### Materials

Materials: coenzyme Q10, gelatin, phospholipid, and glyceryl caprylate-caprate
Equipment: homogenizer, high-performance liquid chromatography (HPLC), spray dryer, particle size analyzer, pH meter, water bath, instability coefficient tester, constant temperature oscillator
All of the above materials are conventional and commercially available.

### Preparation Method

(1) the amounts of coenzyme Q10, glyceryl caprylate-caprate, glyceryl mono- and distearate in a formula were fully dissolved and mixed homogenously to obtain a component labeled as A;
(2) the amounts of phospholipid and gelatin in the formula were fully dissolved in water and mixed homogenously to obtain a component labeled as B;
(3) the component A was poured into the component B, stirred and mixed to form a homogeneous system, followed by twice homogenization at 600 bar;
(4) the homogenized solution was thermally sterilized at a high temperature (80 to 100°C, 10 to 30 min) to obtain a liposome solution; optionally
(5) 20 to 70 parts of an excipient were added into the homogenized solution in the step (3), and the solution added with the excipient was fully homogenized, followed by thermal sterilization at a high temperature (at 80 to 100°C for 10 to 30min). Finally, the moisture is removed by spray drying, freeze drying, and the like, thus obtaining a coenzyme Q10 liposome powder.

### Test Method

### Appearance Stability Evaluation

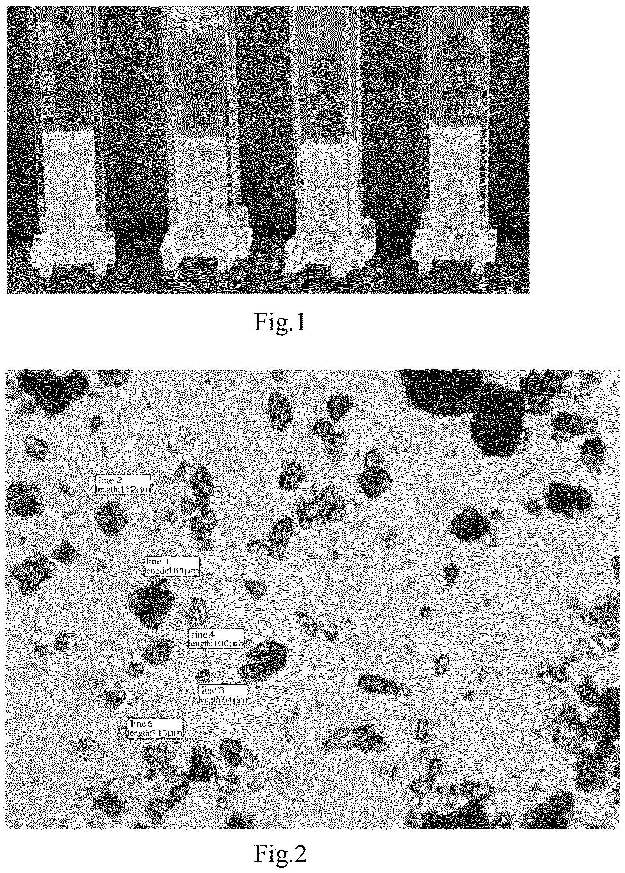

Appearance stability evaluation: The coenzyme Q10 liposome samples (obtained after the step (3)) were sterilized at 90°C for 30 min, and the precipitation or stratification degree was observed. As shown in Fig. 1, the precipitation or stratification degree increases from right to left. Fig. 1 shows a comparison graph of the precipitation or stratification degree after high-temperature sterilization of coenzyme Q10 liposome samples.

The assessment criteria were as follows (see Fig. 1, from left to right):
+++: Obvious stratification or precipitation on the liquid surface after sterilization;
++: Relatively obvious precipitation or stratification on the liquid surface after sterilization;
+: A small amount of precipitation or stratification on the liquid surface after sterilization;
-: No precipitation or stratification on the liquid surface after sterilization.

### Microscopic observation:

The test sample was taken on a glass slide, spread homogeneously, and covered with a cover glass to prepare a temporary mounting slide.

A transflective polarizing microscope (VPV-900) was used. The specific procedures were as follows: adjust the brightness, fix the temporary mounting slide on the stage, align the light aperture with a low-power objective lens, and adjust the coarse focus screw until the field of view is clear. If the clarity is insufficient, use the fine focus screw for further adjustment or switch to a high-power objective lens for observation.

Bright spots can be observed in the middle of the sample in the field of view, which were crystalline structures.

### Electron microscope-scanning observation:

The specific operations for SEM observation were as follows: dip a small amount of powder with a toothpick, sprinkle it homogeneously on the conductive adhesive, blow off the excess powder with a dust removal can, and then perform gold sputtering. The sample was sputter-coated with gold (platinum) using a Quorum SC7620 sputter coater, with the current controlled at 5 to 7 mA and the sputtering time set to 60 s.

After sample preparation, the morphology of the sample was observed using a Zeiss Sigma 300 scanning electron microscopy. Secondary electron imaging was used to record the sample morphology, resulting in the SEM image of the sample.

### Particle size and stability test:

The prepared coenzyme Q10 liposome samples were sterilized at 90°C for 30 min, and then their particle size and stability were measured.

The particle size was measured using a Mastersizer 3000 laser particle size analyzer. The specific operations were as follows: run the system for measuring liposome particle size, wash it three times, add the sample to be measured, start the measurement after the obscuration reaches the system-set value, test each sample three times, and take the average value as the particle size of the coenzyme Q10 liposome. When Dx(50), D[3,2], and D[4,3] were all close, it indicated a uniform particle size distribution of the sample.

The stability test was conducted using an instability coefficient tester with the following specific procedures: the system was set to 4000 rpm for 2 hours, the sample was placed into the system, and the system was operated. The stability of the sample was evaluated based on the instability coefficient-the smaller the coefficient, the better the stability. Stratification or precipitation occurred when the instability coefficient of a coenzyme Q10 liposome sample exceeded 0.2.

### Content determination:

The determination was performed using high-performance liquid chromatography (HPLC) in accordance with General Principle 0512 of the Chinese Pharmacopoeia. The chromatographic conditions and system suitability test were as follows: the chromatographic column was packed with octadecylsilane-bonded silica gel; methanol-absolute ethanol (1:1) was used as the mobile phase; the column temperature was set at 35°C; and the detection wavelength was 275 nm. An appropriate amount of coenzyme Q10 reference substance and coenzyme Q9 reference substance were weighed, dissolved, and diluted with anhydrous ethanol to prepare a mixed solution containing approximately 0.2 mg of each component per 1 mL. A 20 µL aliquot of this mixed solution was injected into the liquid chromatograph. The resolution between the coenzyme Q9 peak and coenzyme Q10 peak should exceed 4, and the theoretical plate number calculated based on the coenzyme Q10 peak should not be less than 3000. The determination method was as follows. 20 mg of the prepared sample was precisely weighed and transferred to a flask. Approximately 40 mL of absolute ethanol was added, and the mixture was shaken to dissolve in a 50°C water bath. After cooling, the solution was transferred to a 100 mL volumetric flask and diluted to the mark with absolute ethanol, followed by thorough mixing. A 20 µL aliquot of this solution was accurately measured as the test solution and injected into the liquid chromatograph to record the chromatogram. Separately, an appropriate amount of coenzyme Q10 reference substance was determined using the same method. The peak area was calculated by the external standard method to obtain the coenzyme Q10 content (denoted as W1). A standard curve of coenzyme Q10 content versus peak area was plotted, and W1 was calculated by interpolating the peak area obtained from HPLC into the standard curve.

### Retention rate:

After sterilization of coenzyme Q10 liposome samples at 90°C for 30 minutes, an accelerated stability assay was performed for three months at 40°C and 75% humidity. The contents of coenzyme Q10 at 0 month and 3 months were determined according to the method in section "content determination". The retention rate of coenzyme Q10 liposomes was calculated according to the following formula:
Retention rate = detected actual content of coenzyme Q10 (W1)/detected value at 0-month (W0) × 100%.

### Encapsulation efficiency determination:

After sterilization of coenzyme Q10 liposome samples at 90°C for 30 minutes, an accelerated stability assay was performed for three months at 40°C and 75% humidity. The encapsulation efficiency was determined according to the method in section "content determination".

Free coenzyme Q10: 0.2 g of the sample after the accelerated stability test was weighed, and 2 mL of n-hexane was added. The mixture was vortex-shaken for extraction and centrifuged at 10,000 rpm for 10 minutes. The upper n-hexane layer was removed, and the determination was performed according to the method in 8.3.3 to obtain the content of free coenzyme Q10 in the liposomes.

Total content of coenzyme Q10: the total content of coenzyme Q10 in coenzyme Q10 liposomes was directly determined according to the determination method in section "content determination". Encapsulation efficiency = (1 - the content of free coenzyme Q10/the total content of coenzyme Q10) × 100%.

### Redissolvability test:

One gram of coenzyme Q10 liposome powder (with a coenzyme Q10 content of 5%) was taken and added to 200 mL of warm water to prepare a solution. Excellent redissolvability was defined as complete dispersion achieved by stirring within 30 seconds without agglomeration or precipitation. The shorter the dissolution time, the better the redissolvability demonstrated.

### In vitro digestion experiments:

Using the INFOGEST in vitro digestion method, the digestion and absorption of coenzyme Q10 liposomes in the body (i.e., bioaccessibility) was evaluated by simulating digestion and absorption in the oral and gastrointestinal tract. The specific operations were as follows: The coenzyme Q10 liposome sample was sterilized at 90°C for 30 minutes, and then a sample equivalent to 50 mg of coenzyme Q10 was weighed. Purified water was added to make the total weight 5 g to prepare the test solution. Simulated salivary fluid (SSF), simulated gastric fluid (SGF), and simulated intestinal fluid (SIF) were prepared by adding corresponding enzymes, namely oral mucosal protein, pepsin, and pancreatin, pancreatic lipase, and bile salt, respectively. The pH of the salivary fluid was adjusted to 6.8, the gastric fluid to 3.0, and the intestinal fluid to 7.0. All were activated at 37.2°C for one hour for later use. The test solution was taken and added to 4 mL of simulated salivary fluid. Oral digestion was carried out at a constant temperature of 37°C with shaking for 10 minutes in a simulated oral digestion environment at pH 6.8. After digestion with simulated salivary fluid, 8 mL of simulated gastric fluid was added to the resulting digested solution. The simulated gastric digestion was performed at a constant temperature of 37°C for 1 hour in a simulated gastric digestion environment at pH 3.0. After digestion with simulated gastric fluid, 16 mL of simulated intestinal fluid was added to the digested solution. Simulated intestinal digestion was conducted for 2 hours in a simulated intestinal digestion environment at pH 7.0, with dynamic pH adjustment to maintain pH at 7.0 during this period, thus completing the simulated intestinal digestion. Finally, the reaction was terminated in an ice bath, and the product was centrifuged at 14,000 rpm for 30 minutes to obtain the supernatant, which was the micelle layer. The pre-digestion content was the actual content of coenzyme Q10 before the in vitro digestion experiment. The contents of coenzyme Q10 before digestion and in the micellar layer were both measured according to the method in the "content determination" section.

**Table 1: Formulas of the digestive juices**

| | | | SSF | | SGF | | SIF | |
|---|---|---|---|---|---|---|---|---|
| Added salt solution | Concentratio n in a stock solution | | Added stock solution in mL, prepare 0.4 L (1.25 x) | Final salt concentratio n in SSF | Added stock solution in mL, prepare 0.4 L (1.25 x) | Final salt concentratio n in SGF | Added stock solution in mL, prepare 0.4 L (1.25 x) | Final salt concentratio n in SIF |
| | (g/L) | (M) | (mL) | (mM) | (mL) | (mM) | (mL) | (mM) |
| KCl | 37.3 | 0.5 | 15.1 | 15.1 | 6.9 | 6.9 | 6.8 | 6.8 |
| KH₂PO₄ | 68 | 0.5 | 3.7 | 3.7 | 0.9 | 0.9 | 0.8 | 0.8 |
| NaCl | 117 | 2 | 3.4 | 13.6 | 18.05 | 72.2 | 30.85 | 123.4 |
| MgCl₂ (H₂O)₆ | 30.5 | 0.15 | 0.5 | 0.15 | 0.4 | 0.12 | 1.1 | 0.33 |
| (NH₄)₂CO₃ | 48 | 0.5 | 0.06 | 0.06 | 0.5 | 0.5 | / | / |
| Concentrated HCl | / | 6 | 0.09 | 1.1 | 1.3 | 15.6 | 0.7 | 8.4 |
| CaCl₂(H₂O)2 | 44.1 | 0.3 | 0.025 | 1.5 | 0.005 | 0.15 | 0.04 | 0.6 |

### Calculation formula:

Bioaccessibility = the content of coenzyme Q10 in micellar layer/the content of coenzyme Q10 before digestion × 100%

### Example 1: Determination of the effect of the Bloom value of gelatin

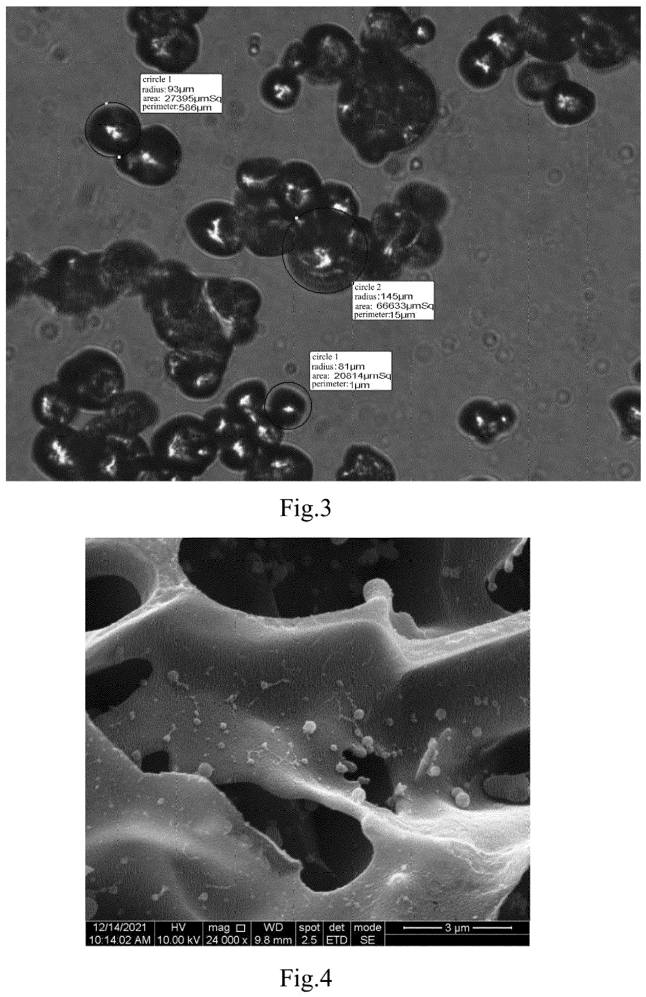

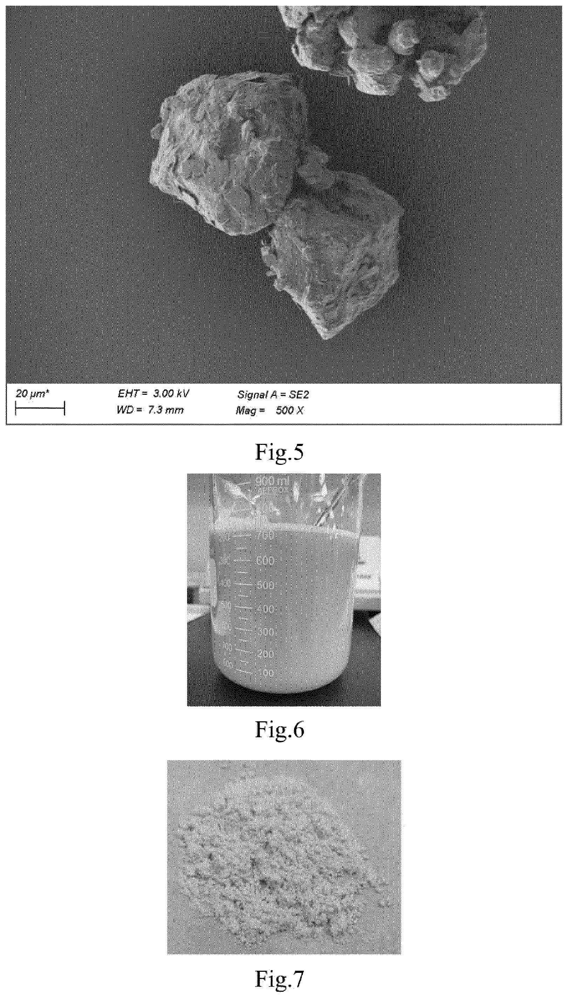

Samples were prepared according to the formulas (in parts by weight) of Table 2, using gelatins with different Bloom values. Subsequently, the particle size, instability coefficient, appearance stability, encapsulation efficiency and retention rate of the coenzyme Q10 liposome samples were evaluated. The results demonstrated that when coenzyme Q10 liposomes were prepared using gelatin with a Bloom value below 200, the resulting liposome products were in an unstable state. Precipitation or stratification occurred after standing; the particle size was large and heterogeneous, and both the retention rate and encapsulation efficiency decreased substantially after three months of accelerated storage. Conversely, coenzyme Q10 liposomes prepared using gelatin with a Bloom value of 200 or greater exhibited enhanced stability. They possessed small and uniform particle sizes, and after three months of accelerated storage, there were no notable alterations in the retention rate and encapsulation efficiency (see Fig. 6). Fig. 4 illustrates the electron microscopy scanning structure of the sample for Composition 6. From the scanning electron microscopy image of the coenzyme Q10 liposome liquid, a large number of circular liposome structures were observable under the electron microscope. Their particle sizes were in accordance with those measured by the particle size analyzer. Fig. 6 depicts the coenzyme Q10 liposome liquid of Composition 6, which appeared as a homogeneous yellow liquid. In summary, not all gelatins are capable of improving liposome stability; gelatins with a high Bloom value can markedly enhance the stability of liposomes.

**Table 2: Liposomes prepared with gelatins with different Bloom values**

| | Composition 1 | Composition 2 | Composition 3 | Composition 4 | Composition 5 | Composition 6 | |
|---|---|---|---|---|---|---|---|
| Gelatin (Bloom value, 150) | 4 | / | / | / | / | / | |
| Gelatin (Bloom value, 180) | / | 4 | / | / | / | / | |
| Gelatin (Bloom value, 200) | / | / | 4 | / | / | / | |
| Gelatin (Bloom value, 220) | / | / | / | 4 | / | / | |
| Gelatin (Bloom value, 250) | / | / | / | / | 4 | / | |
| Gelatin (Bloom value, 300) | / | / | / | / | / | 4 | |
| Phospholipids (PC 30%) | 15 | 15 | 15 | 15 | 15 | 15 | |
| Coenzyme Q10 | 5 | 5 | 5 | 5 | 5 | 5 | |
| Glyceryl caprylate-caprate, | 3 | 3 | 3 | 3 | 3 | 3 | |
| glyceryl mono- and distearate | | 3 | 3 | 3 | 3 | 3 | 3 |
| Water | | 40 | 40 | 40 | 40 | 40 | 40 |
| Particle size (µm) | Dx (50) | 0.383 | 0.161 | 0.273 | 0.263 | 0.265 | 0.260 |
| | D [3,2] | 0.213 | 0.075 | 0.131 | 0.111 | 0.181 | 0.143 |
| | D [4,3] | 1.300 | 0.448 | 0.410 | 0.370 | 0.407 | 0.544 |
| Instability coefficient | | 0.635 | 0.378 | 0.074 | 0.043 | 0.035 | 0.041 |
| Appearance Stability | | +++ | ++ | - | - | - | - |
| Retention rate (%) | | 55.14 | 73.65 | 93.74 | 95.37 | 96.87 | 95.45 |
| Encapsulation efficiency (%) | | 50.12 | 70.34 | 93.25 | 94.34 | 95.56 | 94.21 |

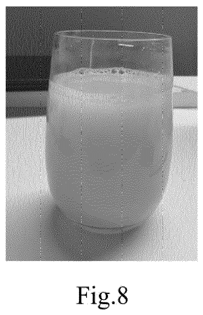

According to the amount of each component of the formulas in Table 2, 30 parts of maltodextrin and 20 parts of isolated whey protein were added. With an inlet air temperature of 100°C and an outlet air temperature of 55°C, spray-drying was employed to remove water and prepare the powder (Compositions 7-12 were obtained using Compositions 1-6, respectively) (see Fig. 7). Subsequently, the redissolvability of each powder was tested, and the results are shown in Table 3. The results indicated that liposome powder prepared with gelatin having a Bloom value of 200 or higher exhibited better redissolvability compared to that prepared with gelatin having a Bloom value below 200 (see Fig. 8).

After microscopic observation of the solution redissolved from Composition 12, the liposomal coenzyme Q10 exhibited a regular spherical crystalline structure under the microscope (Fig. 3). In contrast, the raw material of coenzyme Q10 showed an irregular crystal structure (Fig. 2), indicating the formation of a liposomal structure. Fig. 5 further showed a scanning electron microscopy image of the coenzyme Q10 liposome powder of Composition 12. The scanning electron microscopy (SEM) image of the powdered coenzyme Q10 liposome revealed that the wall material was attached to the liposome surface, presenting an irregular particle structure.

Fig. 7 shows the appearance of the coenzyme Q10 liposome powder of Composition 12. Upon powder preparation, the color of the coenzyme Q10 liposome was lightened by the coating material, presenting as a light-yellow granular powder.

Fig. 8 shows the appearance of the solution redissolved from the coenzyme Q10 liposome powder of Composition 12. Upon dissolution in water, the coenzyme Q10 liposome powder formed a uniform yellow solution.

**Table 3: Effect of the Bloom value of gelatin on the redissolvability of powder**

| | Composition 7 | Composition 8 | Composition 9 | Composition 10 | Composition 11 | Composition 12 |
|---|---|---|---|---|---|---|
| Maltodextrin | 30 | 30 | 30 | 30 | 30 | 30 |
| Isolated whey protein | 20 | 20 | 20 | 20 | 20 | 20 |
| Redissolvability | Partially dissolved with precipitate at the bottom | Partially dissolved with a few of precipitate at the bottom | Completely dissolved within 30s without precipitate | Completely dissolved within 30s without precipitate | Completely dissolved within 30s without precipitate | Completely dissolved within 30s without precipitate |

### Example 2: Determination of the effect of phospholipids with different PC contents

Coenzyme Q10 liposome samples were prepared according to the formulas (in parts by weight) of Table 4, using phospholipids with different PCs. Subsequently, the particle size, instability coefficient, appearance stability, encapsulation efficiency and retention rate of the coenzyme Q10 liposome samples were evaluated.

The results indicated that coenzyme Q10 liposomes prepared with phospholipids of low phosphatidylcholine (PC) content exhibited larger particle size and heterogeneous distribution. Following high-temperature sterilization, particle size increased significantly, accompanied by heavy precipitation or stratification, a high instability coefficient, and low retention rate and encapsulation efficiency after three months of accelerated storage. In contrast, coenzyme Q10 liposomes prepared with phospholipids of higher PC content displayed smaller particle size with homogeneous distribution, no stratification or precipitation upon standing, a lower instability coefficient, and higher retention rate and encapsulation efficiency after three months of acceleration. Notably, when the PC content reached 80%, stability did not improve correspondingly. Thus, optimal stability of coenzyme Q10 liposomes was achieved using phospholipids with moderate PC content (30-60 wt%).

**Table 4: Coenzyme Q10 liposomes prepared with phospholipids with different PC contents**

| | | Composition 13 | Composition 14 | Composition 15 | Composition 16 | Composition 17 |
|---|---|---|---|---|---|---|
| Gelatin (Bloom value, 250) | | 4 | 4 | 4 | 4 | 4 |
| Phospholipid (PC 15%) | | 15 | / | / | / | / |
| Phospholipid (PC 30%) | | / | 15 | / | / | / |
| Phospholipid (PC 45%) | | / | / | 15 | / | / |
| Phospholipid (PC 60%) | | / | / | / | 15 | / |
| Phospholipid (PC 80%) | | / | / | / | / | 15 |
| Coenzyme Q10 | | 5 | 5 | 5 | 5 | 5 |
| Glyceryl caprylate-caprate, | | 3 | 3 | 3 | 3 | 3 |
| glyceryl mono- and distearate | | 4 | 4 | 4 | 4 | 4 |
| Water | | 40 | 40 | 40 | 40 | 40 |
| Particle size (µm) | Dx (50) | 1.86 | 0.265 | 0.400 | 0.300 | 1.255 |
| | D [3,2] | 0.189 | 0.181 | 0.230 | 0.215 | 0.397 |
| | D [4,3] | 1.800 | 0.408 | 0.672 | 0.394 | 1.312 |
| Instability coefficient | | 0.245 | 0.035 | 0.074 | 0.012 | 0.209 |
| Appearance Stability | | ++ | - | - | - | + |
| Retention rate (%) | | 70.38 | 96.87 | 93.76 | 95.97 | 86.85 |
| Encapsulation efficiency (%) | | 67.36 | 95.56 | 92.34 | 94.52 | 85.66 |

According to the amount of each component of the formulas in Table 4, 30 parts of maltodextrin and 20 parts of isolated whey protein were added. With an inlet air temperature of 100°C and an outlet air temperature of 55°C, spray-drying was employed to remove water and prepare the powder (Compositions 18-22 were obtained using Compositions 13-17, respectively). Subsequently, the redissolvability of each powder was tested, and the results were shown in Table 5. The results showed that the liposome powders prepared by using phospholipids with appropriate PC content (30 to 60wt%) had excellent redissolvability.

**Table 5: Effect of phospholipids with different PC contents on powder redissolvability**

| | Composition 18 | Composition 19 | Composition 20 | Composition 21 | Composition 22 |
|---|---|---|---|---|---|
| Maltodextrin | 30 | 30 | 30 | 30 | 30 |
| Isolated whey protein | 20 | 20 | 20 | 20 | 20 |
| Redissolvability | Partially dissolved with precipitate at the bottom | Completely dissolved within 30s without precipitate | Completely dissolved within 30s without precipitate | Completely dissolved within 30s without precipitate | Partially dissolved with a few of precipitate at the bottom |

### Example 3: Determination of the effect of the addition amount of coenzyme Q10 on liposomes

Coenzyme Q10 liposome samples were prepared according to the formulas (in parts by weight) of Table 6, with the content of coenzyme Q10 adjusted. Subsequently, the particle size, instability coefficient, appearance stability, encapsulation efficiency and retention rate of the coenzyme Q10 liposome samples were evaluated.

The results showed that when the coenzyme Q10 ranged from 1 to 7 parts by weight, the prepared coenzyme Q10 liposome showed a small and uniform particle size, excellent stability, and high retention rate and encapsulation efficiency after three months of acceleration. Conversely, when the coenzyme Q10 amount exceeded the 1-7 parts by weight range, the resulting liposomes showed heterogeneous particle size distribution, poor stability, and decreased retention rate and encapsulation efficiency.

**Table 6: Effect of the percentage of coenzyme Q10 on liposomes**

| | | Composition 23 | Composition 24 | Composition 25 | Composition 26 | Composition 27 | Composition 28 |
|---|---|---|---|---|---|---|---|
| Coenzyme Q10 | | 1 | 2 | 3 | 5 | 7 | 8 |
| Phospholipid (PC 30%) | | 15 | 15 | 15 | 15 | 15 | 15 |
| Gelatin (Bloom value, 250) | | 4 | 4 | 4 | 4 | 4 | 4 |
| Glyceryl caprylate-caprate, | | 3 | 3 | 3 | 3 | 3 | 3 |
| glyceryl mono- and distearate | | 4 | 4 | 4 | 4 | 4 | 4 |
| Water | | 40 | 40 | 40 | 40 | 40 | 40 |
| Particle size (µm) | Dx (50) | 0.245 | 0.375 | 0.265 | 0.264 | 0.432 | 1.8 |
| | D [3,2] | 0.124 | 0.18 | 0.181 | 0.214 | 0.241 | 0.212 |
| | D [4,3] | 0.243 | 0.376 | 0.408 | 0.412 | 0.523 | 2.31 |
| Instability coefficient | | 0.013 | 0.024 | 0.035 | 0.047 | 0.056 | 0.841 |
| Appearance Stability | | - | - | - | - | - | ++ |
| Retention rate (%) | | 97.85 | 97.89 | 97.87 | 96.87 | 93.85 | 84.78 |
| Encapsulation efficiency (%) | 97.35 | 96.92 | 96.56 | 95.56 | 92.67 | 77.86 | |

### Example 4: Determination of the effect of the addition amount of phospholipid on coenzyme Q10 liposomes

Coenzyme Q10 liposome samples were prepared according to the formulas (in parts by weight) of Table 7, with the content of phospholipid adjusted. Subsequently, the particle size, instability coefficient, appearance stability, encapsulation efficiency and retention rate of the coenzyme Q10 liposome samples were evaluated.

The results showed that, when the content of phospholipid ranged from 5 to 30 parts by weight, the prepared coenzyme Q10 liposome showed a smaller and uniform particle size, excellent stability, and high retention rate and encapsulation efficiency. Conversely, when the phospholipid content exceeded a specific percentage, precipitation readily occurred, leading to solution stratification.

**Table 7: Effect of different phospholipid percentages on coenzyme Q10 liposomes**

| Formulas | | Composition 29 | Composition 30 | Composition 31 | Composition 32 | Composition 33 | Composition 34 |
|---|---|---|---|---|---|---|---|
| Coenzyme Q10 | | 5 | 5 | 5 | 5 | 5 | 5 |
| Phospholipid (PC 30%) | | 4 | 5 | 10 | 20 | 30 | 31 |
| Gelatin (Bloom value, 250) | | 4 | 4 | 4 | 4 | 4 | 4 |
| Glyceryl caprylate-caprate, | | 3 | 3 | 3 | 3 | 3 | 3 |
| glyceryl mono- and distearate | | 4 | 4 | 4 | 4 | 4 | 4 |
| Water | | 40 | 40 | 40 | 40 | 40 | 40 |
| Particle size (µm) | Dx (50) | 1.8 | 0.375 | 0.265 | 0.264 | 0.432 | 1.4 |
| | D [3,2] | 0.262 | 0.18 | 0.181 | 0.214 | 0.241 | 0.212 |
| | D [4,3] | 2.31 | 0.621 | 0.408 | 0.312 | 0.523 | 1.31 |
| Instability coefficient | | 0.243 | 0.045 | 0.035 | 0.034 | 0.056 | 0.212 |
| Appearance Stability | | +++ | - | - | - | - | + |
| Retention rate (%) | | 50.67 | 93.57 | 96.87 | 96.75 | 96.12 | 90.57 |
| Encapsulation efficiency (%) | | 45.34 | 92.86 | 95.56 | 95.52 | 95.53 | 85.86 |

### Example 5: Determination of the effect of the addition amount of gelatin on coenzyme Q10 liposomes

Coenzyme Q10 liposomes were prepared according to the formulas (in parts by weight) of Table 8, with the content of gelatin adjusted. Subsequently, the particle size, instability coefficient, appearance stability, encapsulation efficiency and retention rate of the coenzyme Q10 liposome samples were evaluated.

The results showed that when the content of gelatin ranged from 3 to 10 parts by weight, the prepared coenzyme Q10 liposome showed a small and uniform particle size, excellent stability, and high retention rate and encapsulation efficiency after three months of acceleration. Conversely, when the content of gelatin exceeded the range of 1 to 7 parts by weight, the prepared coenzyme Q10 liposomes showed a heterogeneous distribution of the particle size, a poor stability, and reduced retention rate and encapsulation efficiency after three months of acceleration.

**Table 8: Effect of different percentages of gelatin on coenzyme Q10 liposomes**

| Formulas | | Composition 35 | Compositio n 36 | Compositio n 37 | Compositio n 38 | Compositio n 39 | Compositio n 40 |
|---|---|---|---|---|---|---|---|
| Coenzyme Q10 | | 5 | 5 | 5 | 5 | 5 | 5 |
| Phospholipids (PC 30%) | | 15 | 15 | 15 | 15 | 15 | 15 |
| Gelatin (Bloom value, 250) | | 2 | 3 | 6 | 8 | 10 | 11 |
| Glyceryl caprylate-caprate, | | 3 | 3 | 3 | 3 | 3 | 3 |
| glyceryl mono- and distearate | | 4 | 4 | 4 | 4 | 4 | 4 |
| Water | | 40 | 40 | 40 | 40 | 40 | 40 |
| Particle size (µm) | Dx (50) | 1.82 | 0.365 | 0.262 | 0.281 | 0.476 | 1.6 |
| | D [3,2] | 0.312 | 0.175 | 0.221 | 0.232 | 0.257 | 0.689 |
| | D [4,3] | 2.43 | 0.563 | 0.458 | 0.322 | 0.543 | 2.11 |
| Instability coefficient | | 0.198 | 0.052 | 0.032 | 0.038 | 0.047 | 0.125 |
| Appearance Stability | | +++ | - | - | - | - | + |
| Retention rate (%) | | 60.34 | 93.56 | 96.67 | 95.43 | 93.42 | 80.34 |
| Encapsulation efficiency (%) | | 57.83 | 92.75 | 95.16 | 94.56 | 92.87 | 67.41 |

### Example 6: In vitro digestion experiment

The in vitro digestion experiment method was as described above. The compositions' formulas and experimental results are presented in Table 9. The results demonstrated that, in the in vitro digestion experiment, the bioaccessibility of coenzyme Q10 liposomes was significantly higher than that of the raw materials. Increasing the Bloom value of gelatin, controlling the phosphatidylcholine (PC) content, and optimizing the phospholipid percentage could enhance the bioaccessibility of coenzyme Q10 liposomes to a certain extent.

**Table 9: Results of in vitro digestion experiments**

| | Comparative Example | Composition 41 | Composition 42 | Composition 43 | Composition 44 |
|---|---|---|---|---|---|
| Gelatin (Bloom value, 250) | / | 4 | / | 4 | 4 |
| Gelatin (Bloom value, 300) | / | / | 4 | / | / |
| Phospholipid (PC 30%) | / | 15 | 15 | / | 20 |
| Phospholipid (PC 60%) | / | / | / | 15 | / |
| Coenzyme Q10 | 5 | 5 | 5 | 5 | 5 |
| Glyceryl caprylate-caprate, | / | 3 | 3 | 3 | 3 |
| glyceryl mono- and distearate | / | 3 | 3 | 3 | 3 |
| Water | 40 | 40 | 40 | 40 | 40 |
| Bioaccessibility (%) | 0.056 | 14.29 | 16.12 | 18.34 | 20.41 |
| folds relative to the comparative example | 1 | 255 | 288 | 328 | 364 |

### Example 7: Liposomes of other effective ingredients

Curcumin, a diketone compound, exhibits pharmacological activities including anti-inflammatory, antioxidant, antibacterial, antitumor, hypolipidemic, and choleretic effects. However, its water insolubility, low oil solubility, and rapid in vivo metabolism limit its practical application in daily products. The liposome composition and preparation method disclosed herein are also applicable to curcumin.

Curcumin liposome samples were prepared according to the formulas (in parts by weight) of Table 10. Subsequently, the particle size, instability coefficient, and appearance stability of the curcumin liposome samples were evaluated.

The results indicated that the composition could improve the stability of curcumin liposomes, which showed that the particle size and instability coefficient were reduced, and the appearance was stable without stratification after high-temperature sterilization.

**Table 10: Determination of stability of liposomes of other effective ingredients**

| | | Composition 45 | Composition 46 | Composition 47 | Composition 48 |
|---|---|---|---|---|---|
| Gelatin (Bloom value, 250) | | 4 | / | 4 | 4 |
| Gelatin (Bloom value, 300) | | / | 4 | / | / |
| Phospholipid (PC 30%) | | 15 | 15 | / | 20 |
| Phospholipid (PC 60%) | | / | / | 15 | / |
| Curcumin | | 5 | 5 | 5 | 5 |
| Glyceryl caprylate-caprate, | | 3 | 3 | 3 | 3 |
| glyceryl mono- and distearate | | 3 | 3 | 3 | 3 |
| Water | | 40 | 40 | 40 | 40 |
| Particle size (µm) | Dx (50) | 0.341 | 0.257 | 0.272 | 0.242 |
| | D [3,2] | 0.298 | 0.253 | 0.261 | 0.218 |
| | D [4,3] | 0.467 | 0.398 | 0.418 | 0.368 |
| Instability coefficient | | 0.045 | 0.028 | 0.031 | 0.025 |
| Appearance Stability | | - | - | - | - |

According to the amount of each component of the formulas in Table 10, 30 parts of maltodextrin and 20 parts of isolated whey protein were added. With an inlet air temperature of 150°C and an outlet air temperature of 70°C, spray-drying was employed to remove water and prepare the powder (Compositions 49-52 were obtained using Compositions 45-48, respectively). Subsequently, the redissolvability of each powder was tested, and the results are shown in Table 11. The results showed that curcumin liposome exhibited excellent redissolvability after being prepared into powder using the compositions disclosed herein.

**Table 11: Determination of the redissolvability of curcumin liposome powder**

| | Composition 49 | Composition 50 | Composition 51 | Composition 52 |
|---|---|---|---|---|
| Maltodextrin | 30 | 30 | 30 | 30 |
| Isolated whey protein | 20 | 20 | 20 | 20 |
| Redissolvability | Completely dissolved within 30s without precipitation | Completely dissolved within 30s without precipitation | Completely dissolved within 30s without precipitation | Completely dissolved within 30s without precipitation |

Carotenoids serve as the primary source of vitamin A in the body. As fat-soluble components, they exhibit potent antioxidant effects, along with immune-regulating, eye-protective, anticancer, and anti-aging properties. However, their susceptibility to oxidation and instability under light and heat significantly limit their practical applications. Lutein and astaxanthin, as typical carotenoids, are widely used in conventional foods and health products. The stability of lutein and astaxanthin liposomes can be effectively enhanced by employing the composition's formulas disclosed herein.

Lutein and astaxanthin liposome samples were prepared according to the formulas (in parts by weight) of Table 12. Subsequently, the particle size, instability coefficient, and appearance stability of the lutein and astaxanthin liposome samples were evaluated.

The results indicated that the composition could improve the stability of lutein and astaxanthin liposomes, which showed that the particle size and instability coefficient were reduced, and the appearance was stable without stratification after high-temperature sterilization.

**Table 12: Determination of the stability of lutein and astaxanthin liposomes**

| | | Composition 53 | Composition 54 | Composition 55 | Composition 56 |
|---|---|---|---|---|---|
| Gelatin (Bloom value, 250) | | 4 | / | 4 | / |
| Gelatin (Bloom value, 300) | | / | 4 | / | 4 |
| Phospholipid (PC 30%) | | 15 | 15 | 15 | 15 |
| Lutein | | 5 | 5 | / | / |
| Astaxanthin | | / | / | 5 | 5 |
| Glyceryl caprylate-caprate, | | 3 | 3 | 3 | 3 |
| glyceryl mono- and distearate | | 3 | 3 | 3 | 3 |
| Water | | 40 | 40 | 40 | 40 |
| Particle size (µm) | Dx (50) | 0.351 | 0.227 | 0.372 | 0.232 |
| | D [3,2] | 0.278 | 0.223 | 0.267 | 0.217 |
| | D [4,3] | 0.447 | 0.364 | 0.436 | 0.338 |
| Instability coefficient | | 0.041 | 0.024 | 0.038 | 0.023 |
| Appearance Stability | | - | - | - | - |

According to the amount of each component of the formulas in Table 12, 30 parts of maltodextrin and 20 parts of isolated whey protein were added. With an inlet air temperature of 120°C and an outlet air temperature of 60°C, spray-drying was employed to remove water and prepare the powder (Compositions 57-60 were obtained using Compositions 53-56, respectively). Subsequently, the redissolvability of each powder was tested, and the results are shown in Table 13. The results showed that Lutein and astaxanthin liposome exhibited excellent redissolvability after being prepared into powder using the compositions disclosed herein.

**Table 13: Determination of the redissolvability of Lutein and astaxanthin liposome powder**

| | Composition 57 | Composition 58 | Composition 59 | Composition 60 |
|---|---|---|---|---|
| Maltodextrin | 30 | 30 | 30 | 30 |
| Isolated whey protein | 20 | 20 | 20 | 20 |
| Redissolvability | Completely dissolved within 30s without precipitation | Completely dissolved within 30s without precipitation | Completely dissolved within 30s without precipitations | Completely dissolved within 30s without precipitation |

Omega-3, an unsaturated fatty acid commonly found in deep-sea fishes and certain plants, offers substantial health benefits to humans. It demonstrates superior efficacy in managing diabetes, cardiovascular diseases, inflammation, dementia, depression, Alzheimer's disease, dry eye conditions, and promoting nerve development and health. The primary types of Omega-3 include α-linolenic acid (ALA), eicosapentaenoic acid (EPA), and docosahexaenoic acid (DHA). Algal oil and fish oil are rich sources of Omega-3, particularly DHA. However, unsaturated fatty acids are prone to oxidation, leading to efficacy loss. The stability of algal oil and fish oil liposomes can be effectively enhanced using the composition's formulas disclosed herein.

Algal oil and fish oil liposome samples were prepared according to the formulas (in parts by weight) of Table 14. Subsequently, the particle size, instability coefficient, appearance stability, encapsulation efficiency and retention rate of the DHA algal oil liposome samples were evaluated.

The results indicated that the composition could improve the stability of algal oil and fish oil liposomes, which showed that the particle size and instability coefficient were reduced, and the appearance was stable without stratification after high-temperature sterilization.

**Table 14 Investigation of the stability of algal oil and fish liposomes**

| | | Composition 61 | Composition 62 | Composition 63 | Composition 64 |
|---|---|---|---|---|---|
| Gelatin (Bloom value, 250) | | 4 | / | 4 | / |
| Gelatin (Bloom value, 300) | | / | 4 | / | 4 |
| Phospholipid (PC 30%) | | 15 | 15 | 15 | 15 |
| Algal oil | | 5 | 5 | / | / |
| Fish oil | | / | / | 5 | 5 |
| glyceryl mono- and distearate | | 3 | 3 | 3 | 3 |
| Water | | 40 | 40 | 40 | 40 |
| Particle size (µm) | Dx (50) | 0.325 | 0.237 | 0.367 | 0.236 |
| | D [3,2] | 0.268 | 0.224 | 0.345 | 0.214 |
| | D [4,3] | 0.445 | 0.383 | 0.428 | 0.349 |
| Instability coefficient | | 0.043 | 0.027 | 0.039 | 0.023 |
| Appearance Stability | | - | - | - | - |
| Encapsulation efficiency | | 91.67 | 94.73 | 93.95 | 95.13 |

According to the amount of each component of formulas in Table 14, 30 parts of maltodextrin and 20 parts of isolated whey protein were added. With an inlet air temperature of 150°C and an outlet air temperature of 70°C, spray-drying was employed to remove water and prepare the powder (Compositions 65-68 were obtained using Compositions 61-64, respectively). Subsequently, the redissolvability of each powder was tested, and the results are shown in Table 15. The results showed that algal oil and fish oil liposome exhibited excellent redissolvability after being prepared into powder using the compositions disclosed herein.

**Table 15: Determination of the redissolvability of algal oil and fish oil liposome powder**

| | Composition 65 | Composition 66 | Composition 67 | Composition 68 |
|---|---|---|---|---|
| Maltodextrin | 30 | 30 | 30 | 30 |
| Isolated whey protein | 20 | 20 | 20 | 20 |
| Redissolvability | Completely dissolved within 30s without precipitation | Completely dissolved within 30s without precipitation | Completely dissolved within 30s without precipitation | Completely dissolved within 30s without precipitation |

Liposoluble vitamins, including vitamin A, D, E, and K, are hydrophobic compounds that are readily soluble in lipids and organic solvents and are typically absorbed alongside lipids. The stability of liposomes encapsulating liposoluble substances, such as vitamin A (VA) and vitamin K (VK) liposomes, can be effectively enhanced using the composition's formulas disclosed herein.

VA and VK liposome samples were prepared according to the formulas (in parts by weight) of Table 16. Subsequently, the particle size, instability coefficient, appearance stability, and encapsulation efficiency of the liposome samples were evaluated.

The results indicated that the composition could improve the stability of VA and VK liposomes, which showed that the particle size and instability coefficient were reduced, and the appearance was stable without stratification after high-temperature sterilization.

**Table 16 Determination of the stability of VA and VK liposomes**

| | | Composition 69 | Composition 70 | Composition 71 | Composition 72 |
|---|---|---|---|---|---|
| Gelatin (Bloom value, 250) | | 4 | / | 4 | / |
| Gelatin (Bloom value, 300) | | / | 4 | / | 4 |
| Phospholipid (PC 30%) | | 15 | 15 | 15 | 15 |
| VA | | 5 | 5 | / | / |
| VK | | / | / | 5 | 5 |
| Glyceryl caprylate-caprate, | | 3 | 3 | 3 | 3 |
| glyceryl mono- and distearate | | 3 | 3 | 3 | 3 |
| Water | | 40 | 40 | 40 | 40 |
| Particle size (µm) | Dx (50) | 0.365 | 0.282 | 0.368 | 0.276 |
| | D [3,2] | 0.308 | 0.244 | 0.315 | 0.234 |
| | D [4,3] | 0.452 | 0.392 | 0.468 | 0.325 |
| Instability coefficient | | 0.041 | 0.023 | 0.040 | 0.019 |
| Appearance Stability | | - | - | - | - |
| Encapsulation efficiency | | 92.42 | 94.46 | 93.24 | 95.02 |

According to the amount of each component of formulas in Table 16, 30 parts of maltodextrin and 20 parts of isolated whey protein were added. With an inlet air temperature of 160°C and an outlet air temperature of 75°C, spray-drying was employed to remove water and prepare the powder (Compositions 73-76 were obtained using Compositions 69-72, respectively). Subsequently, the redissolvability of each powder was tested, and the results are shown in Table 17. The results showed that VA and VK liposomes exhibited excellent redissolvability after being prepared into powder using the compositions disclosed herein.

**Table 17: Determination of the redissolvability of algal oil liposome powder**

| | Composition 73 | Composition 74 | Composition 75 | Composition 76 |
|---|---|---|---|---|
| Maltodextrin | 30 | 30 | 30 | 30 |
| Isolated whey protein | 20 | 20 | 20 | 20 |
| Redissolvability | Completely dissolved within 30s without precipitation | Completely dissolved within 30s without precipitation | Completely dissolved within 30s without precipitation | Completely dissolved within 30s without precipitation |

Water-soluble vitamins, a class of vitamins including vitamin B and vitamin C, are soluble in water but insoluble in non-polar organic solvents. Polyphenols, a large family of naturally occurring phenolics, include epigallocatechin gallate (EGCG), tea polyphenols, etc., and are characterized by multiple phenolic structural units. VC and EGCG are relatively unstable; however, the stability of their liposomes-such as VC liposomes and EGCG liposomes-can be effectively enhanced using the composition's formulas disclosed herein.

VC and EGCG liposome samples were prepared according to the formulas (in parts by weight) of Table 18. Subsequently the particle size, instability coefficient, appearance stability, encapsulation efficiency, and retention rate of the VC and EGCG liposome samples were evaluated.

The results indicated that the compositions could improve the stability of VC and EGCG liposomes, which showed that the particle size and instability coefficient were reduced, and the appearance was stable without stratification after high-temperature sterilization.

**Table 18: Determination of the stability of liposomes of other effective ingredients**

| | | Composition 77 | Composition 78 | Composition 79 | Composition 80 |
|---|---|---|---|---|---|
| Gelatin (Bloom value, 250) | | 4 | / | 4 | / |
| Gelatin (Bloom value, 300) | | / | 4 | / | 4 |
| Phospholipid (PC 30%) | | 15 | 15 | 15 | 15 |
| VC | | 5 | 5 | / | / |
| EGCG | | / | / | 5 | 5 |
| Glyceryl caprylate-caprate, | | 3 | 3 | 3 | 3 |
| glyceryl mono- and distearate | | 3 | 3 | 3 | 3 |
| Water | | 40 | 40 | 40 | 40 |
| Particle size (µm) | Dx (50) | 0.351 | 0.271 | 0.362 | 0.247 |
| | D [3,2] | 0.258 | 0.242 | 0.335 | 0.231 |
| | D [4,3] | 0.468 | 0.353 | 0.452 | 0.351 |
| Instability coefficient | | 0.047 | 0.032 | 0.049 | 0.033 |
| Appearance Stability | | - | - | - | - |
| Encapsulation efficiency | | 71.58 | 82.69 | 73.35 | 85.63 |

According to the amount of each component of the formulas in Table 18, 30 parts of maltodextrin and 20 parts of isolated whey protein were added. With an inlet air temperature of 140°C and an outlet air temperature of 60°C, spray-drying was employed to remove water and prepare the powder (Compositions 81-84 were obtained using Compositions 77-80, respectively). Subsequently, the redissolvability of each powder was tested, and the results are shown in Table 19. The results showed that VC and EGCG liposomes exhibited excellent redissolvability after being prepared into powder using the compositions disclosed herein.

**Table 19: Determination of the redissolvability of VC and EGCG liposome powder**

| | Composition 81 | Composition 82 | Composition 83 | Composition 84 |
|---|---|---|---|---|
| Maltodextrin | 30 | 30 | 30 | 30 |
| Isolated whey protein | 20 | 20 | 20 | 20 |
| Redissolvability | Completely dissolved within 30s without precipitation | Completely dissolved within 30s without precipitation | Completely dissolved within 30s without precipitation | Completely dissolved within 30s without precipitation |

While the present disclosure has been described in conjunction with illustrative embodiments, those skilled in the art will recognize that various alterations, deletions, and/or additions may be made, and that elements of the described embodiments may be substituted with equivalents, all without deviating from the disclosure's spirit and scope. Moreover, numerous modifications can be adapted to specific contexts or materials in accordance with the disclosure's teachings, without exceeding its scope. Therefore, this disclosure is not intended to be limited to the particular embodiments disclosed for implementing it; rather, it is intended to encompass all embodiments falling within the scope of the appended claims.

## Claims

1. A liposome composition comprising 2 to 5 parts by weight of a liquid oil, 3 to 10 parts by weight of a solid oil, 5 to 30 parts by weight of a phospholipid, 3 to 10 parts by weight of gelatin, 1 to 7 parts by weight of an active ingredient, and 20 to 70 parts by weight of water.

2. The liposome composition according to claim **1,** wherein the liquid oil is one or more of glyceryl caprylate-caprate, soybean oil, and sunflower seed oil; and/or
the solid oil is one or more of glyceryl mono- and distearate, citrate, palm stearin, and beeswax; and/or
the phospholipid is a phospholipid with a phosphatidylcholine content of 30 wt% to 60 wt%; and/or
the gelatin is gelatin with a Bloom value between 200 and 300; and/or the liposome composition does not comprise an excipient or comprises 20 to 70 parts by weight of an excipient.

3. The liposome composition according to claim 1 or 2, wherein the active ingredient is selected from the group consisting of a fat-soluble active ingredient, a water-soluble active ingredient, a heat-sensitive active ingredient, an insoluble particle, an acid-sensitive active ingredient, or a digestive enzyme-sensitive active ingredient.

4. The liposome composition according to claim 3, wherein the active ingredient is one or more of coenzyme Q10, curcumin, lutein, astaxanthin, algal oil and fish oil, a vitamin, and a polyphenol.

5. The liposome composition according to claim **1,** wherein the liposome composition does not comprise one or more of the following ingredients: cholesterol, ethanol, and Tween.

6. The liposome composition according to claim 2, wherein the excipient is one or more of a colloid, a maltodextrin, a cyclodextrin, a starch, a sugar alcohol, a syrup, and a whey protein.

7. The liposome composition according to claim 4, wherein the vitamin is selected from the group consisting of vitamin A, vitamin B, vitamin C, vitamin D, vitamin E, and vitamin K; and the polyphenol is selected from the group consisting of epigallocatechin gallate (EGCG) and tea polyphenol.

8. A method of preparing a liposome from the liposome composition of any one of claims 1-7, comprising mixing the liquid oil, the solid oil, the phospholipid, the gelatin, the active ingredient, and water to form a liposome fluid.

9. The method according to claim 8, wherein the active ingredient is a fat-soluble active ingredient or a water-soluble active ingredient;
when the fat-soluble active ingredient is used, the method comprises:
(1) mixing homogeneously the fat-soluble active ingredient, the liquid oil, and the solid oil to obtain a component labeled as A1;
(2) mixing homogeneously the phospholipid, the gelatin, and water to obtain a component labeled as B1; and
(3) mixing the component A1 and the component B1 to form a homogeneous liquid system;
or when the water-soluble active ingredient is used, the method comprises:
(1) mixing homogeneously the liquid oil and the solid oil to obtain a component labeled as A2;
(2) mixing homogeneously the water-soluble active ingredient, the phospholipid, the gelatin, and water to obtain a component labeled as B2; and
(3) mixing the component A2 and the component B2 to form a homogeneous liquid system.

10. A method of preparing a dosage form of a liposome, comprising the method according to claim 8, and further comprising one or more of the following steps:
thermally sterilizing the liposome liquid to prepare a sterilized liquid liposome;
adding a colloid excipient to the liposome liquid, sufficiently dispersing the colloid excipient, followed by thermal sterilization to obtain a liposome gel; or
adding an excipient to the liposome liquid, followed by mixing, thermal sterilization and drying to obtain a liposome powder; wherein the excipient is one or more of maltodextrin, cyclodextrin, starch, sugar alcohol, syrup, and whey protein.

11. A liposome liquid prepared by the method according to claim 8.

12. A liposome dosage form prepared from the liposome fluid according to claim 11, wherein the liposome dosage form is a solution, a gel, a powder, a tablet, a soft capsule, or a gummy.

13. A liposome dosage form, which is the liquid liposome, the liposome gel, or the liposome powder prepared by the method according to claim 10.

14. Use of the liposome dosage form of claim 12 in food or health product or in the manufacture of a medicament.
